# EUROPEAN PATENT SPECIFICATION

(11) **EP 2 464 235 B1**
(45) Date of publication and mention of the grant of the patent: **12.07.2017**
(21) Application number: 10808781.8
(22) Date of filing: 12.08.2010
(51) Int. Cl.: A01N 59/16, A01N 37/36, A01N 25/34, A01P 1/00, C09D 5/14, A61L 2/23

(54) **FORMULATIONS AND METHODS EMPLOYING ANHYDROUS DISINFECTANT**
FORMULIERUNGEN UND VERFAHREN MIT EINEM WASSERFREIEN DESINFEKTIONSMITTEL
FORMULATIONS ET PROCEDES UTILISANT UN DESINFECTANT ANHYDRE

(30) Priority: 12.08.2009 US 233424 P
(43) Date of publication of application: 20.06.2012
(73) Proprietor: Pure Bioscience, El Cajon, CA 92020 (US)
(72) Inventor: KRALL, Michael, L., El Cajon CA 92020 (US); JONTE, Dolana, El Cajon CA 92020 (US); GUMIENNY, Richard, El Cajon CA 92020 (US)
(74) Representative: Uexküll & Stolberg
(86) International application number: PCT/US2010/045369
(87) International publication number: WO 2011/019951

(56) References cited:
- EP-A1- 1 839 645
- WO-A2-2005/041861
- US-A1- 2005 245 605
- US-A1- 2006 115 440
- US-A1- 2006 254 988
- US-A1- 2007 185 350
- US-A1- 2008 156 232
- US-A1- 2008 156 232
- US-A1- 2008 319 062
- US-A1- 2009 035 342
- "Opinion on Citric acid (and) Silver citrate", , 21 January 2009 (2009-01-21), pages 1-30, XP55042238, Retrieved from the Internet: URL:http://ec.europa.eu/health/ph_risk/com mittees/04_sccp/docs/sccp_o_165.pdf [retrieved on 2012-10-25]
- Hannu Viitanen ET AL: "Improved Model to Predict Mold Growth in Building Materials", Buildings X, 1 January 2007 (2007-01-01), pages 1-8, XP55064854, Retrieved from the Internet: URL:http://www.ornl.gov/sci/buildings/2012 /Session PDFs/162_New.pdf [retrieved on 2013-05-31]
- M. Bar ET AL: "Modelling the survival of bacteria in drylands: the advantage of being dormant", Proceedings of the Royal Society B: Biological Sciences, vol. 269, no. 1494, 7 May 2002 (2002-05-07), pages 937-942, XP55064874, ISSN: 0962-8452, DOI: 10.1098/rspb.2002.1958

## Description

### Technical Field

The invention relates to the non-therapeutic use of the anhydrous form of silver dihydrogen citrate (SDC) directly, without reconstitution in aqueous liquid, for antimicrobial protection. More specifically, the invention relates to non-therapeutic direct use of SDC in non-aqueous compositions.
The invention also relates to a composition comprising SDC for use in applying to a wound an antimicrobially effective amount without reconstitution in an aqueous solution.

### Background Art

The preparation of aqueous solutions of silver dihydrogen citrate (SDC) and citric acid was described in U.S. 6,197,814. In this method, the SDC is generated electrolytically in a solution of 5-25% citric acid. According to the '814 patent, the resulting aqueous disinfectant may optionally be mixed with alcohol and/or a detergent and can be used on exposed or contaminated surfaces to kill bacteria, virus, fungi and other microorganisms. It can also be used to disinfect wounds and to behave as a disinfectant in water systems, such as cooling towers, hot water systems, potable water systems, etc.

US 2006/254988 describes a composition comprising silver citrate at a concentration greater than 4000ppm, preferably in the form of silver dihydrogen citrate.

PCT publication WO2005/041861 and U.S. patent 7,732,486, describes preparing an anhydrous form of the SDC/citric acid solution for ease of transport. According to this publication, in order to employ the anhydrous form as a disinfectant, it should be reconstituted to recreate the aqueous systems described in the '814 patent. As noted in the '861 publication and the '486 patent, the anhydrous form is capable of being reconstituted to a fully-active aqueous disinfectant.

It has now been found that reconstitution of this anhydrous form is unnecessary, and that the anhydrous form itself may be employed in non-aqueous systems as an *in situ* disinfectant and antimicrobial protectant.

### Disclosure of the Invention

In one aspect, the present invention provides a solid formulation wherein an antimicrobial amount of an anhydrous composition of silver dihydrogen citrate and citric acid (citric acid/SDC composition) is included in the formulation wherein the weight percentage of the anhydrous composition in the formulation is from about 0.5% by weight to about 50% by weight, and wherein the solid formulation is selected from the group consisting of a medical device coating; a plastic; fiberglass material; woven or nonwoven fabric; a polymer; vinyl; high pressure laminate; paper/cardboard packaging; construction material; drywall; drywall finishing compounds; tile; tile grout, a combination with anti-clotting agents to provide anti-infective properties; a combination with medical powders to be applied as an anti-infective dusting agent for pre- and post-surgical procedures or trauma wound application; a combination with covalent dry chemistry for use as a biological warfare decontaminant; a bandage substrate; and a combination with filter medium to mitigate microbial growth.

In a further aspect the present invention provides a method to provide antimicrobial protection to a surface, which method comprises applying to said surface an antimicrobially effective amount of an anhydrous composition of silver dihydrogen citrate (SDC) and citric acid (anhydrous citric acid/SDC composition) without reconstitution of said composition in an aqueous solution wherein the surface is selected from the group consisting of the surfaces of plant; ornamental and cut flowers; carpeting; bandages; and upholstery.

In order to avoid confusion, the anhydrous SDC/citric acid itself will be referred to as a "composition" and a solid or non-aqueous liquid formulation in which it has been included will be referred to as a "formulation."

### Modes of Carrying Out the Invention

A method for obtaining anhydrous SDC/citric acid compositions is described in detail in the above-referenced PCT publication WO2005/01861 and U.S. 7,732,486. Briefly, as therein disclosed, an aqueous solution of SDC in the presence of significant amounts of citric acid is prepared generally according to the process set forth in U.S. 6,197,814, *i*.*e*., the SDC is generated by applying either DC or AC current across silver electrodes immersed in a citric acid solution containing, for example, citric acid at a concentration of the order of 1-25% generally 5-20% or 5-10% by weight.

The water is removed from the resulting aqueous disinfectant, preferably by freeze-drying a frozen solution under vacuum effecting sublimation of the water. Other means for removing water from an appropriate SDC/citric acid solution may also be employed, such as vacuum drying, spray drying, or other means of effecting removal of the aqueous solvent so long as the antimicrobial effect of the SDC/citric acid composition is not destroyed.

The anhydrous composition may be milled into suitable particle sizes for application. Any art-recognized means for disaggregating and fluidizing solids may be employed. Typical particle sizes range from 2-500 µm, but larger or smaller sizes may also be employed. For medical use, for example, the particles maybe in the range of 100-1,000 nm.

"Antimicrobial" includes effective protection against fungi, viruses, bacteria, archaea. Thus, any infectious agent is included as a "microbial" agent.

"Non-aqueous formulations" refers to formulations that effectively exclude any water component whatsoever, or that include only 5%-15% water by weight or less. They do not include emulsions, lotions, and the like that have substantial amounts of water but rather either are liquid formulations, wherein no more than 5-15% preferably no more than 1-5% by weight is water, or are solid materials of any type. Emulsions and lotions that contain SDC/citric acid are described in PCT publication WO2006/029213. However, these formulations are prepared directly from the aqueous solutions of SCD/citric acid. The present invention also includes methods of preparing these compositions or similar compositions using the anhydrous composition *per se.*

In some embodiments, the anhydrous composition or a formulation containing it ultimately comes in contact with an aqueous environment; in other applications, it does not. In each case, however, the anhydrous SDC/citric acid composition is employed without reconstituting it first into an aqueous solution.

In exemplary applications, the anhydrous composition is included in various coatings and construction materials, such as paints or other coatings or materials employed to construct solid components such as countertop materials including Formica^{®} and high pressure laminates, drywall, plaster and tile. The use of this material in drywall is especially important to prevent the formation of mold. The anhydrous composition may also directly be used in packaging materials such as paper, cardboard, Styrofoam™, plastics. It may also be used in caulks and sealants as well as in woven and non-woven fabrics including synthetic and natural fabrics.

Depending on the nature of the application, the weight percentage of the anhydrous composition in the finished product will vary over a wide range from about 0.5% by weight to about 50% by weight, typically 1-2% by weight. The anhydrous composition may be incorporated in the manufacture of the product, for example, by mixing the composition directly into paint or varnish or by including it in a preparation of monomers to be polymerized in the formation of polymers or in the particulates pressed into fiberboard. The SDC/citric acid anhydrous composition is compatible with a wide variety of materials and skilled artisans will understand how best to incorporate this composition effectively into non-aqueous systems.

The dried SDC/citric acid composition of the invention is also useful in the context of medical and pharmaceutical applications and can be applied as a dry coating to bandages and medical devices, such as catheters or surgical tools. The dry SDC/citric acid may also be directly applied to wounds, especially wherein deep wounds, internal wounds or subcutaneous infections are a threat. The dry SDC/citric acid can be combined with various other pharmaceuticals in tablets, powders. Other pharmaceuticals include antibiotics, anti-clotting agents, and effervescing systems.

Anhydrous SDC/citric acid compositions of the invention are also useful directly in water treatment by integrating them with filter media, and employed in solid form in various contexts such as toilet bowl sanitizers, and are also useful in agriculture for crop-dusting either alone or in combination with other dried materials such as fertilizers and herbicides and pesticides.

A list of such uses is as follows:
Anhydrous SDC combined with an acceptable substrate to provide antimicrobial protection in commercial processes:
   Plastics and fiberglass materials (FRP systems)
      Woven and nonwoven fabrics: organic and inorganic; filament and monolithic strand Polymers
   Vinyls
   Paint: protective coating systems - single and multi-part, *e.g.* to prevent mold in drywall Latex
   Architectural finishes, i.e. : Formica®/countertop materials and other high pressure laminates
      Caulks and conforming sealants
      Hardening Foams, thermoformed and catalyzed
      Paper/cardboard packaging and attendant coatings
   Construction materials: drywall and drywall finishing compounds, plaster, tile and tile grouts
   Anhydrous SDC for medical and pharmaceutical applications:
      Combined with antibiotics as a synergizing/potentiating agent
      Combined with anti-clotting agents to provide anti-infective properties
      Combined with medical powders to be applied as an anti-infective dusting agent for pre and post surgical procedures or trauma wound application
      Combines with co-valent dry chemistry for use as a biological warfare decontaminant Incorporated into a bandage substrate
      Tabletized chemistries: effervescing and non-effervescing systems utilizing other actives and excipients
      Gel & semi-solid suppository delivery systems
      Medical device coatings
      Curative / palliative treatment of subcutaneous and deeper internal infections

### Water Treatment:

Combined with water treatment resins
Combined with filter media to mitigate microbial growth
Introduced into fluid handling systems for biofilm mitigation
Aquaculture

### Agriculture:

Systemic treatment for plant pathogens utilizing various time-release methodologies Soil pH moderator
Dry sanitizer for pre-and post-harvest rinse
Tabletized unit dosing for process equipment
Unit dose ornamental & cut flower preservative

### Commercial Mass Market:

Dry formed tablet / pelletized home fruit and vegetable wash
Tabletized unit dose home hard surface sanitizer
Toilet sanitizer
Unit dose carpet and upholstery sanitizer systems

The following examples are offered to illustrate the invention.

### Example 1

### Preparation of Antimicrobial Drywall

Antimicrobial drywall is prepared by incorporating silver dihydrogen citrate (SDC) in the gypsum core, in or on the paper covering, or in both the gypsum core and paper covering of drywall. The SDC-containing drywall resists the growth of microbes, in particular fungi, when compared to standard drywall.

Antimicrobial drywall containing SDC in the gypsum core is prepared by adding solid SDC to a slurry containing gypsum powder, water, paper pulp, starch and/or set controlling agents, in an amount sufficient to exhibit efficacy against microbes. The resultant gypsum slurry is sandwiched between two sheets of craft paper stock, referred to as the front and back paper facings. The paper with the slurry in between is run through a set of rollers to make it flat and smooth, the edges are shaped, and the drywall is cut to the desired size and dried.

Antimicrobial drywall containing SDC in the paper covering is prepared by spraying one or both of the front and back paper facings with a solution of SDC in an appropriate solvent prior to drying the cut drywall. Alternatively, SDC is applied as a coating on one or both sides of the paper covering before the paper is contacted with the gypsum slurry.

### Example 2

### Reparation of Antimicrobial Paper

A slurry of paper forming fibers prepared according to a wet-laid process is mixed with an aluminum silicate or titanium dioxide filler in a quantity of about 1%-10% parts per weight of fiber. The mixture is stirred constantly with the addition of starch paste as a sizing compound. An interfiber binding agent is prepared by adding solid SDC to latex polyacrylamide or polyvinyl acetate, as a melt or as a solution in a compatible solvent, and mixing to incorporate the SDC within the polymeric matrix. The binding agent mixture is added to the pulp material and mixed, and the mixture is poured onto a wire screen, dewatered, pressed and calendared to obtain a smooth finish.

### Example 3

### Reparation of Antimicrobial Grout

Silver dihydrogen citrate (SDC) is admixed with a standard dry grout compound in an amount sufficient to exhibit efficacy against microbes.

The dry admixture is mixed with water according to packaging instructions and cast into 1-2 inch diameter rounds for testing. The SDC-containing samples resist *Aspergillus niger* (household black mold) relative to control samples lacking SDC when exposed to simulated household shower conditions.

### Example 4

### Preparation of Antimicrobial High Pressure Laminate

SDC is admixed into a conventional melamine formulation, including ceramic reinforcement materials and pigments, in an amount sufficient to exhibit efficacy against microbes. The melamine mixture is spread onto craft paper and dried. The SDC-containing melamine layers are pressed together using conventional heating and pressing equipment to produce a high-pressure laminate material.

The SDC-containing laminate material resists the growth of microbes, including bacteria and fungi, relative to traditional laminates.

### Example 5

### Preparation of Antimicrobial Coated Catheters

An antimicrobial coating composition is prepared by admixing finely ground silver dihydrogen citrate (SDC) with a hexane dispersion of room temperature vulcanized (RTV) silicone resin. The surface of the catheter to be coated is cleaned, and the article is dip-coated by immersing in the SDC-containing mixture for several minutes. The catheter is dipped into the solution 1 to 3 times, until the desired thickness of the coating is achieved. The article is allowed to dry between coatings and then further dried at elevated temperature.

The treated catheter resists the growth of undesirable microbes relative to an untreated control when incubated at 37°C for 24 to 48 hours with inoculum cultures of test bacteria, such as E. *coli* (*e*.*g*., clinical isolate from UTI).

## Claims

1. A solid formulation wherein an antimicrobial amount of an anhydrous composition of silver dihydrogen citrate and citric acid (citric acid/SDC composition) is included in the formulation wherein the weight percentage of the anhydrous composition in the formulation is from about 0.5% by weight to about 50% by weight, and wherein the solid formulation is selected from the group consisting of a medical device coating; a plastic; fiberglass material; woven or nonwoven fabric; a polymer; vinyl; high pressure laminate; paper/cardboard packaging; construction material; drywall; drywall finishing compounds; tile; tile grout, a combination with anti-clotting agents to provide anti-infective properties; a combination with medical powders to be applied as an anti-infective dusting agent for pre- and post-surgical procedures or trauma wound application; a combination with covalent dry chemistry for use as a biological warfare decontaminant; a bandage substrate; and a combination with filter medium to mitigate microbial growth.

2. The solid formulation of claim 1 wherein said anhydrous citric acid/SDC composition is in the form of microparticles.

3. The solid formulation of claim 2 wherein the microparticles have average diameters of 2-500 µm.

4. The solid formulation of any of claims 1-3 wherein the citric acid in said anhydrous citric acid/SDC composition is in at least 5-fold molar excess of the SDC.

5. The solid formulation of any of claims 1-4 wherein said solid formulation is a construction material, a solid polymer, a paper product, or a plastic.

6. A method to provide antimicrobial protection to a surface, which method comprises applying to said surface an antimicrobially effective amount of an anhydrous composition of silver dihydrogen citrate (SDC) and citric acid (anhydrous citric acid/SDC composition) without reconstitution of said composition in an aqueous solution wherein the surface is selected from the group consisting of the surfaces of plant; ornamental and cut flowers; carpeting; bandages; and upholstery.

7. The method of claim 6, wherein the citric acid in said anhydrous citric acid/SDC composition is in at least 5-fold molar excess of the SDC.

8. The method of claim 6 or 7, wherein said anhydrous citric acid/SDC composition is in the form of microparticles.

9. The method of claim 8 wherein the microparticles have average diameters of 2-500 µm.

10. An anhydrous composition of silver dihydrogen citrate and citric acid (citric acid/SDC composition) for use in applying to a wound an antimicrobial effective amount of said composition without reconstitution of said composition in an aqueous solution.

11. A method to provide antimicrobial protection to a formulation which method comprises including in said formulation an antimicrobial effective amount of an anhydrous composition of SDC and citric acid without reconstitution of said composition wherein the solid formulation is selected from the group consisting of a medical device coating; a plastic; fiberglass material; woven or nonwoven fabric; a polymer; vinyl; high pressure laminate; paint; latex; architectural finishes; caulks; conforming sealants; hardening foams; paper/cardboard packaging; cementitious coatings; drywall; drywall finishing compounds; tile; tile grout, a combination with anti-clotting agents to provide anti-infective properties; a combination with medical powders to be applied as an anti-infective dusting agent for pre- and post-surgical procedures or trauma wound application; a combination with covalent dry chemistry for use as a biological warfare decontaminant; a bandage substrate; and a combination with filter medium to mitigate microbial growth.

## Patentansprüche

1. Feste Formulierung, wobei die Formulierung eine antimikrobiell wirksame Menge einer wasserfreien Zusammensetzung aus Silber Dihydrogen Citrat und Zitronensäure (Zitronensäure/SDC-Zusammensetzung) enthält, wobei der Gewichtsprozentsatz der wasserfreien Zusammensetzung in der Formulierung etwa 0,5 Gew .-% bis etwa beträgt 50 Gew .-%, und wobei die feste Formulierung aus folgender Gruppe aus ausgewählt wurde: Beschichtung einer medizinischen Vorrichtung; Kunststoff; Fiberglas-Material; gewebtem oder nicht gewebtem Stoff; Polymer; Vinyl; Hochdrucklaminat; Papier-/Kartonverpackung; Baumaterial; Trockenbauwand; Trockenbau-Oberflächenverbindungen; Fliesen; Fliesenmörtel, Kombination mit Gerinnungshemmstoffen, um antiinfektiöse Eigenschaften zu erhalten; Kombination mit medizinischen Pulvern, die als anti-infektiöse Staubmittel bei prä- und postoperativen Verfahren oder bei der traumatischen Wundbehandlung angewendet werden; Kombination mit kovalenter Trockenchemie zur Dekontamination biologischer Kampfmittel; Verbandsubstrat; Kombination mit Filtermedium, um das mikrobielle Wachstum zu mildern.

2. Feste Formulierung gemäß Anspruch 1, wobei die wasserfreie Zitronensäure / SDC-Zusammensetzung in Form von Mikropartikeln vorliegt.

3. Feste Formulierung gemäß Anspruch 2, wobei die Mikropartikel einen mittleren Durchmesser von 2-500 µm aufweisen.

4. Feste Formulierung gemäß einem der Ansprüche 1 bis 3, wobei die Zitronensäure in der wasserfreien Zitronensäure/SDC-Zusammensetzung in mindestens 5-fachem molaren Überschuss in Verhältnis zum SDC liegt.

5. Feste Formulierung gemäß einem der Ansprüche 1 bis 4, wobei die feste Formulierung ein Baumaterial, ein festes Polymer, ein Papierprodukt oder ein Kunststoff ist.

6. Verfahren zur Herstellung eines antimikrobiellen Schutzes auf einer Oberfläche, wobei das Verfahren das Aufbringen einer antimikrobiell wirksamen Menge einer wasserfreien Zusammensetzung aus Silber Dihydrogen Citrat (SDC) und Zitronensäure (wasserfreie Zitronensäure/SDC-Zusammensetzung) ohne Rekonstitution der Zusammensetzung in einer wässrigen Lösung auf der Oberfläche umfasst, wobei die Oberfläche aus folgender Gruppe ausgewählt ist: Oberfläche einer Pflanze; Zier- und Schnittblumen; Teppichboden; Bandagen; und Polsterung.

7. Verfahren gemäß Anspruch 6, **dadurch gekennzeichnet, dass** die Zitronensäure in der wasserfreien Zitronensäure/SDC-Zusammensetzung in mindestens 5-fachem molaren Überschuss im Verhältnis zum SDC vorliegt.

8. Verfahren gemäß Anspruch 6 oder 7, wobei die wasserfreie Zitronensäure/SDC-Zusammensetzung in Form von Mikropartikeln vorliegt.

9. Verfahren gemäß Anspruch 8, **dadurch gekennzeichnet, dass** die Mikropartikel einen mittleren Durchmesser von 2-500 µm aufweisen.

10. Wasserfreie Zusammensetzung aus Silber Dihydrogen Citrat und Zitronensäure (Zitronensäure/SDC-Zusammensetzung) zur Verwendung bei der Verabreichung einer antimikrobiell wirksamen Menge der Zusammensetzung auf eine Wunde ohne Rekonstitution der Zusammensetzung in einer wässrigen Lösung.

11. Verfahren zur Herstellung eines antimikrobiellen Schutzes für eine Formulierung, wobei das Verfahren Schritte umfasst, bei denen man eine antimikrobiell wirksame Menge einer wasserfreien Zusammensetzung aus SDC und Zitronensäure ohne Rekonstitution der Zusammensetzung in die Formulierung aufnimmt, wobei die feste Formulierung aus folgender Gruppe ausgewählt ist: Beschichtung einer medizinischen Vorrichtung; Kunststoff; Fiberglas-Material; gewebter oder nicht-gewebter Stoff; Polymer; Vinyl; Hochdrucklaminat; Farbe; Latex; architektonische Oberflächen; Dichtungen; konforme Dichtstoffe; härtende Schaumstoffe; Papier- oder Kartonverpackungen; Zement-haltige Beschichtungen; Trockenbauwand; Trockenbau-Oberflächenverbindungen; Fliesen; Fliesenmörtel; Kombination mit Gerinnungshemmstoffen, um antiinfektiöse Eigenschaften zu erhalten; Kombination mit medizinischen Pulvern, die als anti-infektiöse Staubmittel bei prä- und postoperativen Verfahren oder bei der traumatischen Wundbehandlung angewendet werden; Kombination mit kovalenter Trockenchemie zur Dekontamination biologischer Kampfmittel; Verbandsubstrat; Kombination mit Filtermedium, um das mikrobielle Wachstum zu mildern.

## Revendications

1. Formulation solide dans laquelle une quantité antimicrobienne d'une composition anhydre de dihydrogénocitrate d'argent et d'acide citrique (composition de DCA/acide citrique) est incluse dans la formulation, le pourcentage en poids de la composition anhydre dans la formulation valant d'environ 0,5 % en poids à environ 50 % en poids, et la formulation solide étant choisie dans l'ensemble constitué par un revêtement de dispositif médical ; une matière plastique ; un matériau à base de fibres de verre ; un tissu tissé ou non-tissé ; un polymère ; le vinyle ; un stratifié haute pression ; un emballage en papier/carton ; un matériau de construction ; une cloison sèche ; des composés de finition de cloison sèche ; un carreau ; un joint de carrelage, une association avec des agents anticoagulants pour conférer des propriétés anti-infectieuses ; une association avec des poudres médicales à appliquer en tant qu'agent de poudrage anti-infectieux pour des procédures pré- et post-chirurgicales ou application sur des blessures ; une association avec chimie sèche covalente pour utilisation en tant que décontaminant d'arme biologique ; un substrat de pansement ; et une association avec un milieu filtrant pour restreindre une croissance bactérienne.

2. Formulation solide selon la revendication 1, dans laquelle ladite composition anhydre de DCA/acide citrique est sous la forme de microparticules.

3. Formulation solide selon la revendication 2, dans laquelle les microparticules ont des diamètres moyens de 2-500 µm.

4. Formulation solide selon l'une quelconque des revendications 1 à 3, dans laquelle l'acide citrique dans ladite composition anhydre de DCA/acide citrique présente un excès au moins 5 fois molaire du DCA.

5. Formulation solide selon l'une quelconque des revendications 1 à 4, ladite formulation solide étant un matériau de construction, un polymère solide, un produit à base de papier ou une matière plastique.

6. Procédé pour fournir une protection antimicrobienne à une surface, lequel procédé comprend l'application sur ladite surface d'une quantité à efficacité antimicrobienne d'une composition anhydre de dihydrogénocitrate d'argent (DCA) et d'acide citrique (composition anhydre de DCA/acide citrique) sans reconstitution de ladite composition en une solution aqueuse, dans lequel la surface est choisie dans l'ensemble constitué par les surfaces d'un végétal ; de fleurs ornementales et de fleurs coupées ; de moquettes ; de pansements ; et de tissus d'ameublement.

7. Procédé selon la revendication 6, dans lequel l'acide citrique dans ladite composition anhydre de DCA/acide citrique présente un excès au moins 5 fois molaire du DCA.

8. Procédé selon la revendication 6 ou 7, dans lequel ladite composition anhydre de DCA/acide citrique est sous la forme de microparticules.

9. Procédé selon la revendication 8, dans laquelle les microparticles ont des diamètres moyens de 2-500 µm.

10. Composition anhydre de dihydrogénocitrate d'argent et d'acide citrique (composition de DCA/acide citrique) pour utilisation dans l'application sur une plaie d'une quantité à efficacité antimicrobienne de ladite composition sans reconstitution de ladite composition en une solution aqueuse.

11. Procédé pour fournir une protection antimicrobienne à une formulation, lequel procédé comprend l'inclusion dans ladite formulation d'une quantité à efficacité antimicrobienne d'une composition anhydre de DCA et d'acide citrique sans reconstitution de ladite composition, ladite formulation solide étant choisie dans l'ensemble constitué par un revêtement de dispositif médical ; une matière plastique ; un matériau à base de fibres de verre ; un tissu tissé ou non-tissé ; un polymère ; le vinyle ; un stratifié haute pression ; une peinture ; un latex ; des finitions architecturales ; des calfeutrages ; des agents de scellement conformateurs ; des mousses durcissantes, un emballage en papier/carton ; des revêtements cimentaires ; une cloison sèche ; des composés de finition de cloison sèche ; un carreau ; un joint de carrelage, une association avec des agents anticoagulants pour conférer des propriétés anti-infectieuses ; une association avec des poudres médicales à appliquer en tant qu'agent de poudrage anti-infectieux pour des procédures pré- et post-chirurgicales ou application sur des blessures ; une association avec chimie sèche covalente pour utilisation en tant que décontaminant d'arme biologique ; un substrat de pansement ; et une association avec un milieu filtrant pour restreindre une croissance bactérienne.
